# EUROPEAN PATENT SPECIFICATION

(11) **EP 1 633 440 B1**
(45) Date of publication and mention of the grant of the patent: **14.05.2008**
(21) Application number: 04727664.7
(22) Date of filing: 15.04.2004
(51) Int. Cl.: A61P 7/02, A61P 25/28

(54) **PHARMACEUTICAL COMPOSITION COMPRISING PROTEINS AND/OR POLYPEPTIDES AND COLLOIDAL PARTICLES**
PHARMAZEUTISCHE ZUSAMMENSETZUNG, ENTHALTEND PROTEINE UND/ODER POLYPEPTIDE SOWIE KOLLOIDPARTIKEL
COMPOSITION PHARMACEUTIQUE CONTENANT DES PROTEINES ET/OU DES POLYPEPTIDES ET DES PARTICULES COLLOIDALES

(30) Priority: 15.04.2003 US 462701 P
(43) Date of publication of application: 15.03.2006
(73) Proprietor: OPPERBAS HOLDING B.V., 1102 BR Amsterdam Zuidoost (NL)
(72) Inventor: BARU, Moshe, 37000 Pardes Hana (IL); CARMEL-GOREN, Lea, IL-52324 Ramat Gan (IL)
(74) Representative: Minoja, Fabrizio
(86) International application number: PCT/IL2004/000327
(87) International publication number: WO 2004/091723

(56) References cited:
- WO-A-98/32466
- WO-A-99/55306
- WO-A-02/086117
- US-A- 5 013 556

## Description

### BACKGROUND OF THE INVENTION

Hemophilia A is one of the most frequently occurring inherited coagulation disorders. Patients with hemophilia A are prone to frequent hemorrhages as a result of one or more misfunctions of the coagulation system. One of the causes of hemophilia is a shortage of Factor VIII (FVIII) in the blood. This problem can be treated with Factor VIII concentrates. However, in about 15% of the patients the occurrence results of Factor VIII neutralizing antibodies, so-called inhibitors, whereby a therapy with Factor VIII concentrates is hardly possible.

Two basic approaches have been described in the literature to protect FVIII from inactivation by inhibitors.

WO/80/01456 to Hemker discloses a pharmaceutical composition suitable for oral administration comprising FVIII incorporated within liposomes of 0.5-1.0 microns formed from phospholipids. The phospholipids have a net charge, and the FVIII is incorporated between the layers of the liposome. It is claimed that FVIII levels in the plasma remained above about 5% of the normal value for a period of 50 hours.

US 4,348,384 to Horikoshi states that a composition as described in Hemker was prepared, but did not give satisfactory results. Therefore, Horikoshi incorporates a protease inhibitor into the liposome together with FVIII, in order to protect it from proteolysis. 3% of the normal plasma levels of FVIII were obtained over a period of 6 hours.

US 5,013,556 to Woodle discloses a liposome composition for use in delivering various drugs via the bloodstream. The liposome contains between 1-20 mole percent of an amphipathic lipid derivatized with a polyalkylether. Here also, the drug compound is entrapped within the liposome. These liposome compositions are available commercially under the name of Stealth^{®} vesicles (SUV's, small unilamellar vesicles comprised of phospholipid and polyethylene glycol (PEG) covalently bound to phospholipid).

A further problem with this approach is that liposomes having a large diameter have a short half-life. Therefore, the liposomes must be downsized under high pressure, which can affect protein activities as in coagulation factors V and VIII.

In a second approach, Barrowcliffe, T.W., et al. (1983) J. Lab. Clin. Med. 101:34-43 teaches that mixing FVIII with phospholipid extracted from human and/or animal brain imparts significant protection to the FVIIII *in vitro.* In this approach, the phospholipid is bound to the FVIII rather than encapsulating it. Kemball-Cook, G. and Barrowcliffe, T.W. (1992) Thromb. Res. 67:57-71, teaches that a negatively-charged phospholipid surface is necessary for FVIII binding. Negatively charged phosphatidyl serine and phophatidic acid were found to be highly active in binding to FVIII, while phosphatidyl choline was inactive. However, negatively-charged phospholipids are toxic, and those derived from brain tissue may carry pathogenic agents.

EP 689,428 discloses a liposome composition comprising liposomes having an outer surface layer of hydrophilic polymer chains. A polypeptide or polysaccharide effector molecule is covalently attached to the distal ends of the polymer chains by activation of the lipid anchor prior to effector coupling.

### SUMMARY OF THE INVENTION

It is an object of the present invention to provide a pharmaceutical composition comprising a protein or polypeptide for therapeutic treatment.

It is a fluther object of the invention to provide a protein or polypeptide in a form having an extended half-life in the bloodstream.

In one aspect of the invention, there is provided a pharmaceutical composition for parenteral administration comprising a therapeutically effective amount of a protein or polypeptide and colloidal particles, said particles comprising approximately 1-20 mole percent of an amphipathic lipid derivatized with a biocompatible hydrophilic polymer, wherein said protein or polypeptide is selected from the group consisting of (a) proteins or polypeptides capable of externally binding said colloidal particles; (b) proteins or polypeptides capable of binding polymers of the polyalkylether, polylactic and polyglycolic acid families; and (c) proteins or polypeptides that include a consensus sequence of B/T-X-LJIfV-I/V/Q/S-S/T-X-X-E, where X may be any amino acid, and S, T, L, I, V, E and Q have their standard meanings. The protein or polypeptide is not encapsulated in said colloidal particles.

In a preferred embodiment, the colloidal particles are substantially neutral and the polymer carries substantially no net charge. In the present specification, the terms *"substantially neutral* and *"substantially no net charge"* mean neither positively nor negatively charged. However, a very low measured charge within experimental error of zero is included within the meaning of the above terms.

The present invention is based on the surprising and unexpected finding that liposomes containing amphipathic lipids derivatized with a bio-compatible hydrophilic polymer can be used to bind proteins or polypeptides, enhance their pharmacokinetic parameters (half-life and area under the curve) and protect them from inhibitors in the bloodstream. This provides a significant advantage over the prior art compositions, since the amphipathic lipids used are synthetic and non toxic, and can therefore be used *in vivo* for therapeutic treatment. Furthermore, the liposome does not encapsulate the proteins or polypeptides so that smaller sized liposomes can be used which have a longer half-life *in vivo,* since they are not removed by the reticuloendothelial system (RES) and the activity of the formulated proteins or polypeptides is not impaired (full activity found *in vitro* and immediately after injection *in vivo*)*.* As will be described below in greater detail, the proteins or polypeptides interact non-covalently with the polymer chains on the external surface of the liposomes, and no chemical reaction is carried out to activate the polymer chains, unlike the composition disclosed in EP 689,428.

The term *"proteins or polypeptides capable of externally binding said colloidal particles"* includes proteins such as coagulation factor VIIa (FVIIa), factor V (FV), factor IX (FIX) and factor X (FX), Granulocyte colony-stimulating factor (G-CSF), Granulocyte macrophage colony-stimulating factor (GM-CSF), Interferon γ and Glucagon-Like Peptide 1 (GLP-1), Interferon-γ and Glucagon-Like Peptide 1 (GLP-1) and a copolymer (Copaxone, Teva, Israel) composed of repeats of 4 amino acids (L-ala, L-glu, L-lys and L-tyr). The identity of these proteins may be ascertained empirically as is known by the skilled man of the art.

The pharmaceutical composition of the invention may be used to treat various diseases, as is known to the skilled man of the art. For example, a composition comprising Copaxone may be used to protect central nervous system (CNS) cells from glutamate toxicity and to treat injury or disease caused or exacerbated by glutamate toxicity. The composition may also be used to treat multiple sclerosis, diabetic neuropathy, senile dementia, Alzheimer's disease, Parkinson's Disease, facial nerve (Bell's) palsy, glaucoma, Huntington's chorea, amyotrophic lateral sclerosis, status epilepticus, non-arteritic optic neuropathy, or vitamin deficiency, as described in US Patent Application No. 20020037848.

The term *"proteins or polypeptides capable of binding polymers of the polyalkylether; polylactic and polyglycolic acid families "* includes proteins and polypeptides which bind to polymers of the polyalkylether, polylactic and polyglycolic acid families or derivatives thereof by any non-covalent mechanism, such as ionic interactions, hydrophobic interactions, hydrogen bonds and Van der Waals attractions (Arakawa, T. and Timasheff, S.N. (1985) Biochemistry 24:6756-6762; Lee, J.C. and Lee, L.L.Y (1981) J. Biol. Chem. 226:625-631). A preferred example of such a polymer is polyethylene glycol (PEG).

The term *"therapeutically effective amount"* is to be understood as referring to an amount of protein or polypeptide which results in a level of the protein or polypeptide in the bloodstream having a desired therapeutic effect. Such an amount can be experimentally determined by administering compositions comprising different amounts of the protein or polypeptide and measuring the level in the blood at various times after administration.

The amphipathic lipid used to prepare the colloidal particles may be obtained from either natural or synthetic sources. Most preferred lipids include phospholipids such as phosphatidylcholine and phosphatidylethanolamine, and carbamate-linked uncharged lipopolymers such as aminopropanediol distearoyl (DS) The purpose of the biocompatible hydrophilic polymer is to sterically stabilize the SUVs, thus preventing fusion of the vesicles *in vitro,* and allowing the vesicles to escape adsorption by the RES *in vivo.* The polymer will preferably have a molecular weight of between about 500 to about 5000 daltons, most preferably approximately 2000 daltons.

The colloidal particles will preferably have a mean particle diameter of between about 0.03 to about 0.4 microns, most preferably about 0.1 microns. This is to increase their circulation time *in vivo* and prevent their adsorption by the RES. The amphipathic lipid comprises approximately 0.5 to about 20 mole % of the particles, preferably approximately 1-6%, most preferably 3%.

A variety of known coupling reactions may be used for preparing vesicle forming lipids derivatized with hydrophilic polymers. For example, a polymer (such as PEG) may be derivatized to a lipid such as phosphatidylethanolamine (PE) through a cyanuric chloride group. Alternatively, a capped PEG may be activated with a carbonyl diimidazole coupling reagent, to form an activated imidazole compound. A carbamate-linked compound may be prepared by reacting the terminal hydroxyl of MPEG (methoxyPEG) with p-nitrophenyl chloroformate to yield a p-nitrophenyl carbonate. This product is then reacted with 1-amino-2,3-propanediol to yield the intermediate carbamate. The hydroxyl groups of the diol are acylated to yield the final product. A similar synthesis, using glycerol in place of 1-amino-2,3-propanediol, can be used to produce a carbonate-linked product, as described in WO 01/05873. Other reactions are well known and are described, e.g. in the aforementioned U.S. 5,013,556.

The composition of the invention may be administered by injection, preferably iv, sc or im. The prior art compositions were intended for oral use only, due to side effects caused during injection by the liposome composition. The composition of the invention, on the other hand, is not toxic by injection, apparently due to the small size and lack of toxic phospholipids. Toxicology studies of PEGylated liposomes and FVIII in rats have been earned out - no toxicity was found at doses of 500 units/kg. Amounts of up to 0.5gm/Kg body weight of colloidal particles according to the invention have been injected without detectable toxic symptoms. Although the free form of the proteins and polypeptides used in the invention have a short half-life in the vascular system, a them in the composition of the invention is expected to increase their half life by at least 1.5 times. The composition of the invention is expected to be effective in "on demand" and prophylactic treatment of patients.

In another aspect of the invention, it has been found that there is a consensus sequence of 8 amino acids located within the proteins that binds the liposomes of the invention but is absent in proteins that do not bind the liposomes. This consensus sequence is S/T-X-LXV-I/V/Q/S-S/T-X-X-E, where X may be any amino acid, and S, T, L, I, V, E and Q have their standard meanings.

In another aspect of the invention, it has been found that injection of PEGylated liposomes separately from the injection of the protein increases the protein half-life and long-term efficacy.

In a further aspect of the invention, cholesterol is supplemented to the pharmaceutical composition.

### BRIEF DESCRIPTION OF THE DRAWINGS

In order to understand the invention and to see how it may be carried out in practice, a preferred embodiment will now be described, by way of examples with reference to the accompanying drawings, in which:

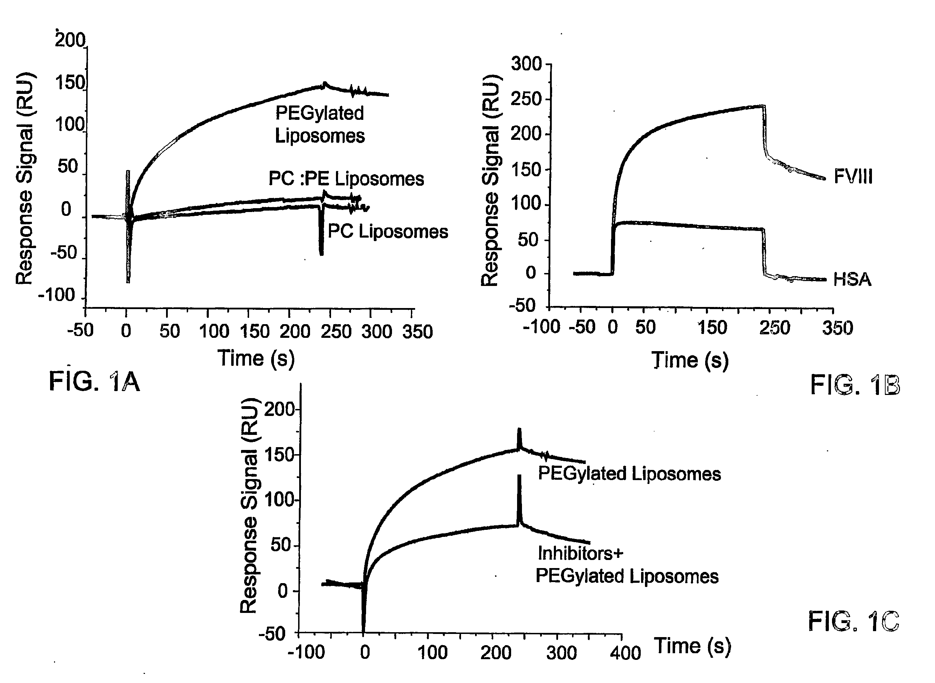

**Fig. 1** illustrates a real time interaction of PEGylated liposomes to immobilized FVIII. *a*, PEGylated liposomes , but not control POPC liposomes (PC) and POPC:DSPE liposomes (PC:PE), bind to immobilized rFVIII. *b*, PEGylated liposomes bind to immobilized rFVIII (FVIII) but not to immobilized HSA . *c*, PEGylated liposomes bind to immobilized rFVIII in the absence or presence (Inhibitors) of a diluted (x100) serum of a hemophilia A patient that developed anti FVIII antibodies (titer of 20 Bethesda units/ml). Response is indicated in Resonance Units (RU). Response in *a-c* is corrected for nonspecific binding to HSA coated channel, which was less than 5°1o Real time interaction of PEGylated liposomes to immobilized FVIII coated channels.

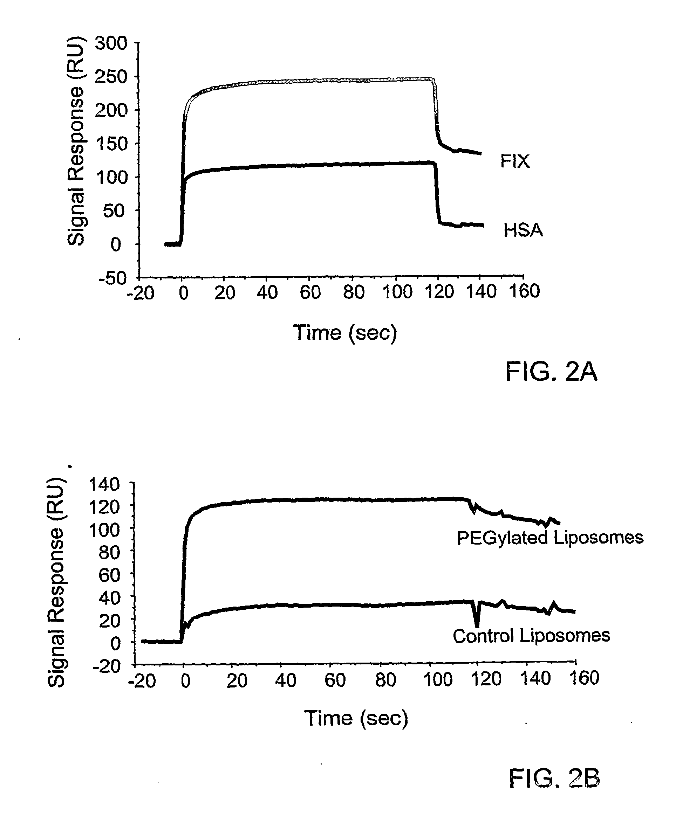

**Fig. 2****.** Real time interaction of PBGylated liposomes to immobilized FIX. a, PEGylated liposomes bind to immobilized FIX but not to immobilized HSA . *b*, PEGylated liposomes , but not control POPC liposomes , bind to immobilized FIX. Response is indicated in Resonance Units (RU). Response is corrected for nonspecific binding to HSA coated channel, which was less than 5% Real time interaction of PEGylated liposomes to immobilized FIX elative to the binding to FIX coated channels.

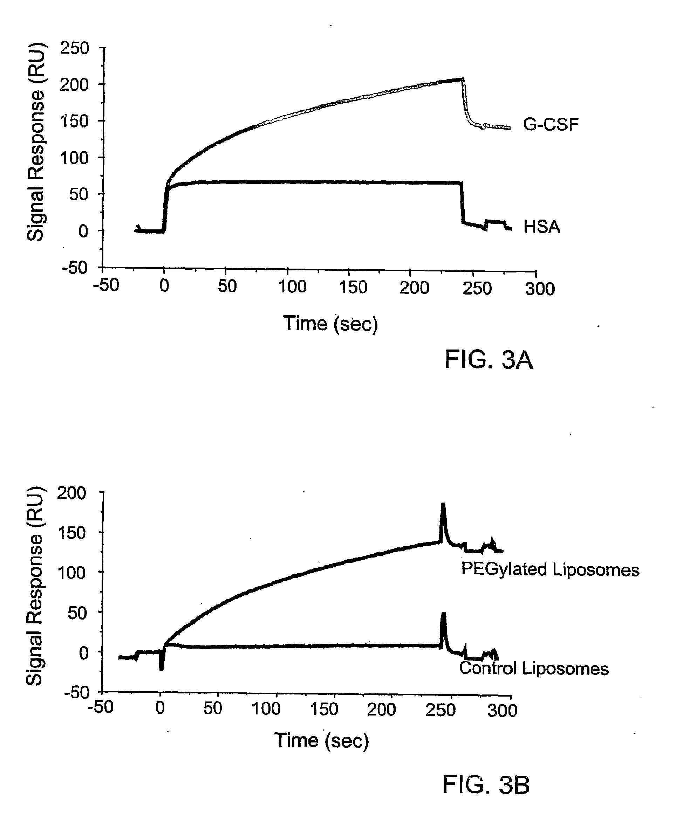

**Fig. 3**. Real time interaction of PEGylated liposomes to immobilized G-CSF. *a*, PEGrylated liposomes bind to immobilized G-CSF but not to immobilized HSA . *b*, PEGylated liposomes , but not control POPC liposomes , bind to immobilized G-CSF. Response is indicated in Resonance Units (RU). Response is corrected for nonspecific binding to HSA coated channel, which was less than 5% Real time interaction of PEGylated liposomes to immobilized G-CSF elative to the binding to G-CSF coated channels.

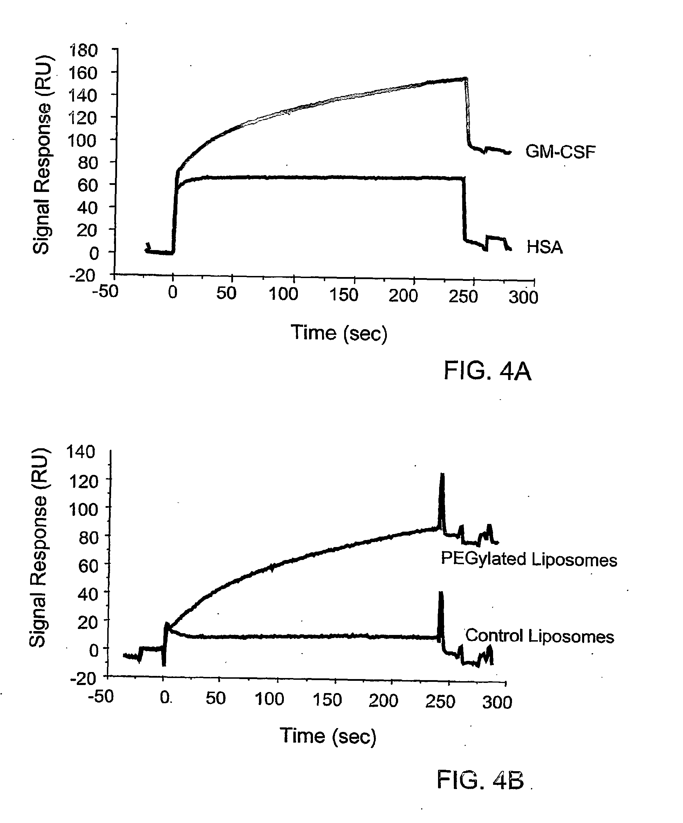

**Fig. 4****.** Real time interaction of PEGylated liposomes to immobilized GM-CSF. *a*, PEGylated liposomes bind to immobilized GM-CSF but not to immobilized HSA . *b*, PEGylated liposomes , but not control POPC liposomes , bind to immobilized GM-CSF. Response is indicated in Resonance Units (RU). Response is corrected for nonspecific binding to HSA coated channel, which was less than 5% Real time interaction of PEGylated liposomes to immobilized GM-CSF elative to the binding to G-CSF coated channels.

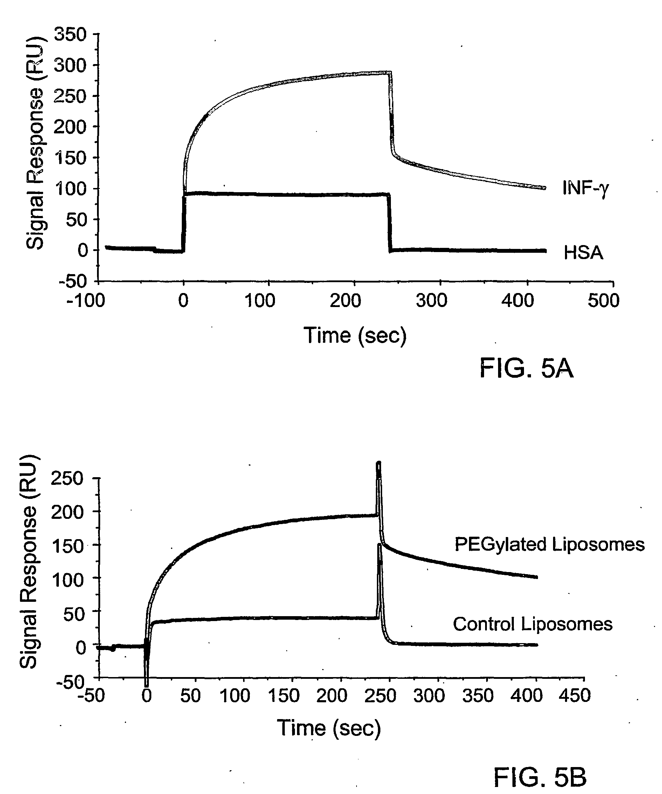

**Fig. 5**. Real time interaction of PEGylated liposomes to immobilized Interferon γ. *a*, PEGylated liposomes bind to immobilized INF-γ but not to immobilized HSA . *b*, PEGylated liposomes , but not control POPC liposomes , bind to immobilized INF-γ. Response is indicated in Resonance Units (RU). Response is corrected for nonspecific binding to HSA coated channel, which was less than 5% Real time interaction of PEGylated liposomes to immobilized INF-γ elative to the binding to INF-γ coated channels.

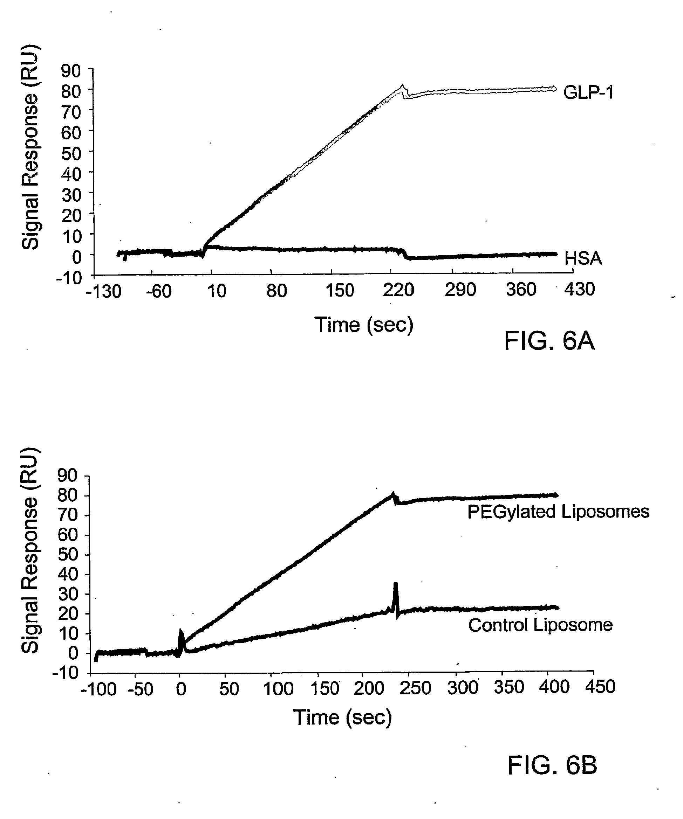

**Fig. 6**. Real time interaction of PEGylated liposomes to immobilized GLP-1. *a*, PEGylated liposomes bind to immobilized GLP-1 but not to immobilized HSA . *b*, PEGylated liposomes , but not control POPC liposomes , bind to immobilized GLP-1. Response is indicated in Resonance Units (RU). Response is corrected for nonspecific binding to HSA coated channel, which was less than 5% Real time interaction of PEGylated liposomes to immobilized GLP-1 elative to the binding to GLP-1 coated channels.

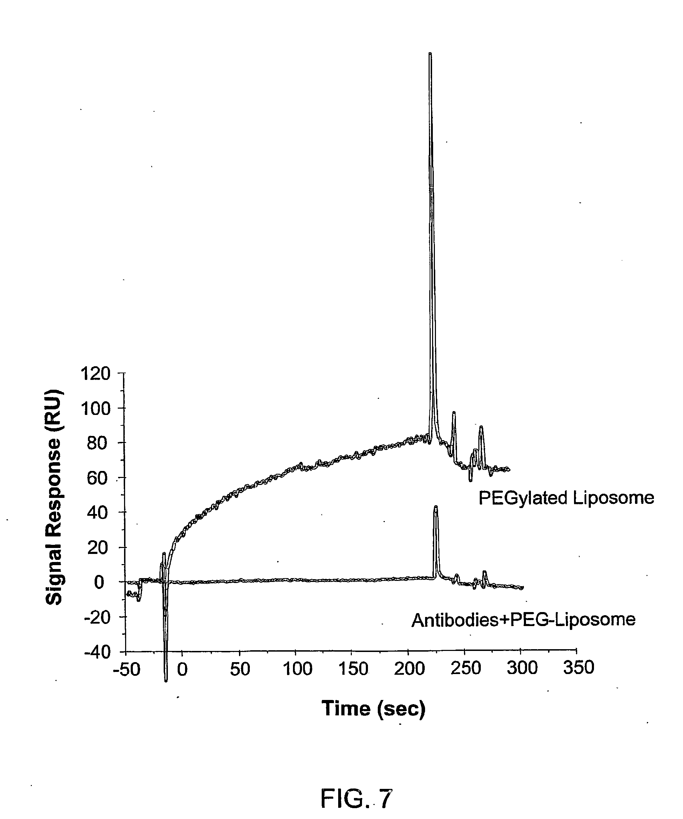

**Fig. 7**. PEGylated liposomes bind to immobilized FIX in the absence or presence Antibodies) of a diluted (x10) rabbit polyclonal anti human FIX antibodies (Sigma).

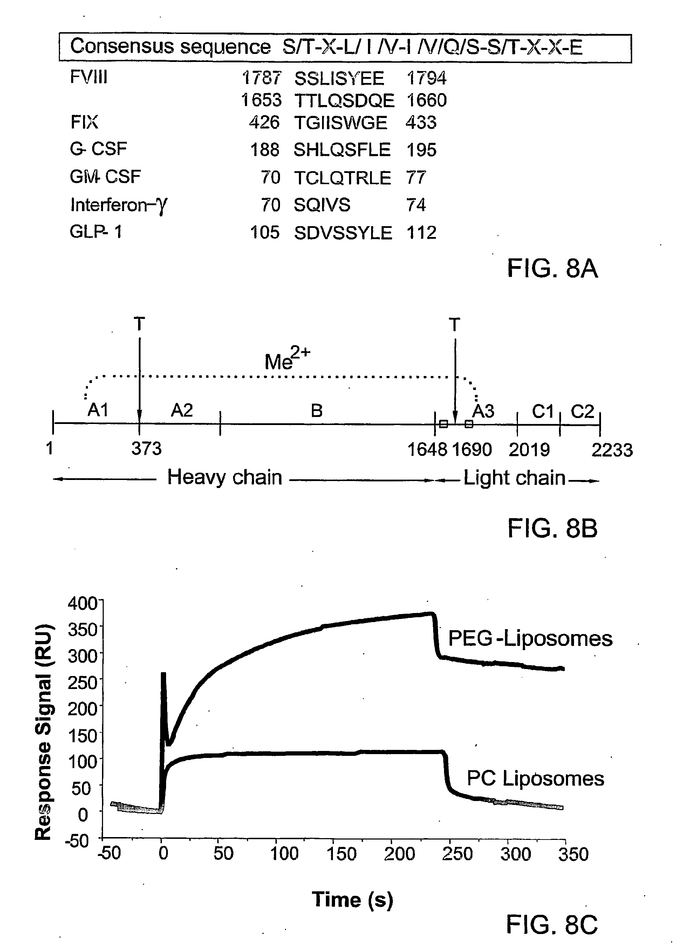

**Fig 8****.** Consensus sequence and FVIII binding sites for PEGylated liposomes. *a*, Consensus sequence for binding PEGylated liposomes in various proteins. Conserved amino acids are depicted in red whereas unconserved amino acids are depicted in blue. *b*, Human FVIII domain structure. Discrete domain structure of human FVIIT as deduced from its primary structure. Liposome binding sites are represented as black squares. Red arrows indicated with T represent thrombin activation sites. *c*, Binding of PEGylated liposomes or POPC liposomes (PC) to a peptide derived from FVIII sequence (1783-1796 amino acids) immobilized at a CM5 sensor chip. Response is indicated in Resonance Units (RU).

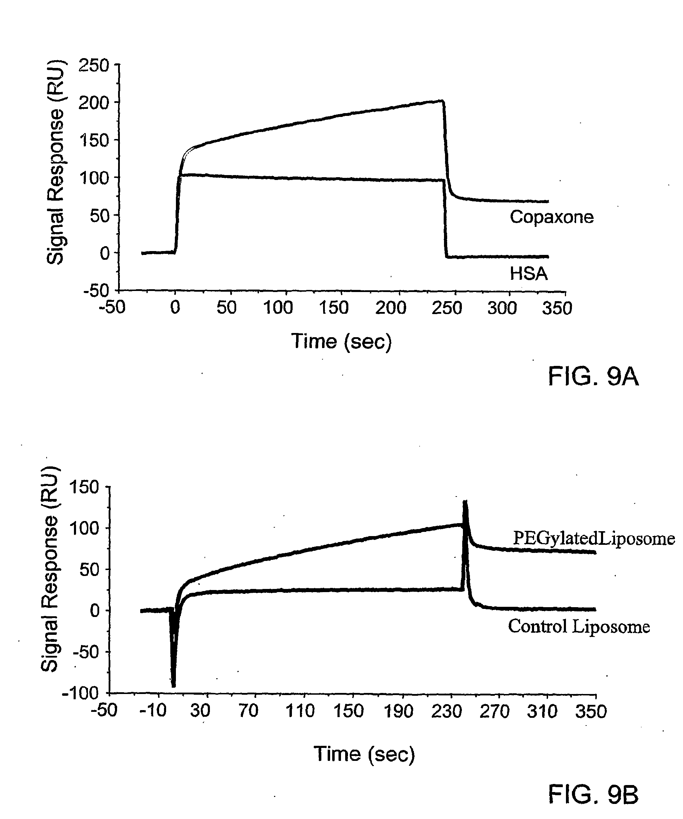

**Fig. 9****.** Real time interaction of PEGylated liposomes to immobilized Copaxone. *a*, PEGylated liposomes bind to immobilized Copaxone but not to immobilized HSA. *b*, PEGylated liposomes, but not control POPC liposomes, bind to immobilized Copaxone. Response is indicated in Resonance Units (RU). Response is corrected for nonspecific binding to HSA coated channel, which was less than 5% Real time interaction of PEGylated liposomes to immobilized Copaxone elative to the binding to Copaxone coated channels.

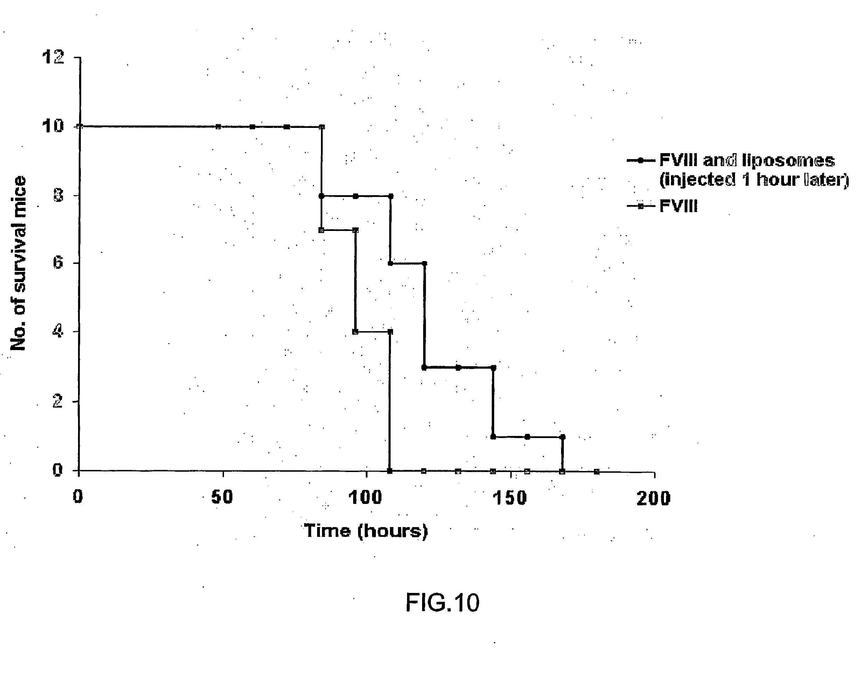

**Fig. 10**. Survival of hemophilic mice following tail cuts. Kaplan-Meierb plots are shown for hemophilic mice injected with FVIII (Kogenate-FS, Bayer) or FVIII and PEGylated liposomes (injected one hour later). T test statistical analysis indicates statistically significant differences between the groups (P<0.05).

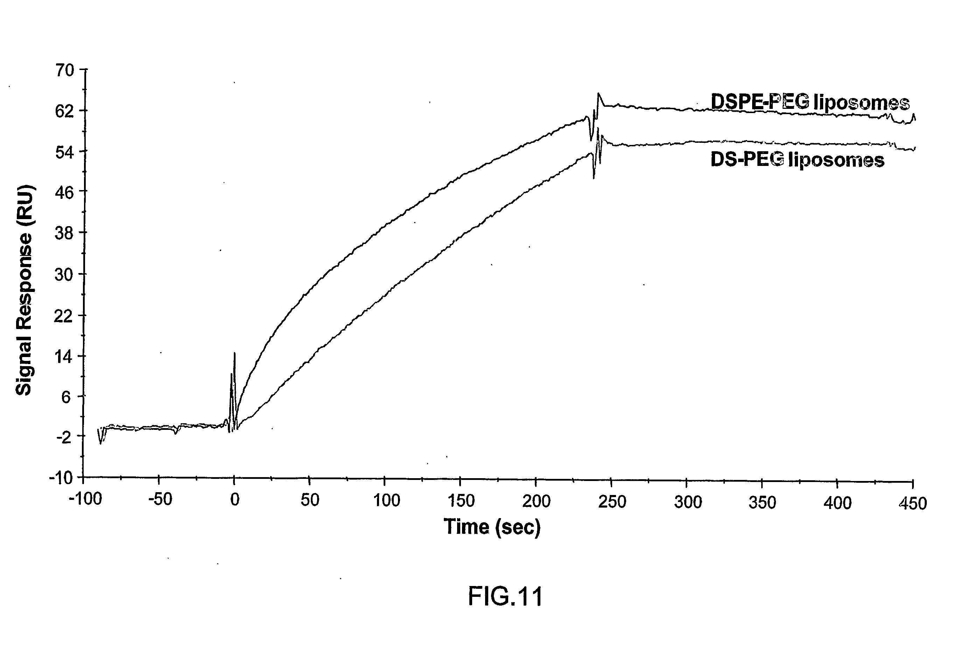

**Fig. 11**. Real time interaction of PEGylated liposomes composed of POPC:DSPE-PEG 2000 (lipids from Genzyme Pharmaceuticals, Liestal, Switzerland) with 97:3 mole ratio, respectively, and PEGylated liposomes composed of POPC:DS-c-PEG2000 (lipids from Genzyme Pharmaceuticals, Liestal, Switzerland) with 97:3 mole ratio, respectively, to immobilized FVIII.

### DETAILED DESCRIPTION OF THE INVENTION

### 1. Examples 1-8

### 1.1 Material & Methods

Liposome preparation. Liposomes composed of pahnitoyl-oleoyl phosphatidyl-choline (POPC) and distearoyl phosphatidyl-ethanolamine methyl polyethylene glycol 2000 (DSPE-PEG 2000) (Genzyme Pharmaceuticals, Liesatal, Switzerland) (97:3 molar ratio, respectively), POPC and distearoyl phosphatidylethanolamine (DSPE) (Aventi Polar Lipids, Alabaster AL, USA) (97:3 molar ratio, respectively) were prepared as follows: lipids were dissolved to 10% w/v in tert-buthanol (Reidel-de Haen, Seelze, Germany), frozen and the solution was lyophilized. The resulting dry lipid powder was re-suspended to 2-13% (w/v) in a 50 mM sodium citrate buffer, pH 7.0 to form liposomes. The liposomes were filtered using LiposoFast^{™}-100 extruder apparatus (Aventin Inc., Ottawa, Canada) through polycarbonate filters 1.2 µm, 0.2 µm, 0.1 µm and 0.05 µm in size (Poetics Corp., Livermore CA, USA) to form liposomes in the size of 80-110 nm. The liposome solution was then passed through a 0.2 µm cellulose acetate sterilizing filter (Sterivex^{™}, Millipore Corporation, Bedford MA, USA) and stored at 2-8 °C.

**Real time interactions -** Surface Plasmon Resonance analysis. Binding studies were performed using a Biacore^{™} 2000 biosensor instrument (Biacore AB, Uppsala, Sweden). The following proteins were immobilized onto a CM5-sensor chip (Biacore AB, Uppsala, Sweden) at 1500RU(~1.5ng/mm²), by the amine coupling kit as prescribed by the supplier: FVIII (Kogenate-FS, Bayer, Berkley CA, USA), FIX (Benefix, Genetics Institute, Cambridge MA, USA), G-CSF, GM-CSF, IFN-γ, Erythropoietin, Human Growth Hormone, Interferon-alpha 2a, Interferon-alpha 2b (ProSpec-Tany TechnoGene Ltd, Nes Ziona, Israel), GLP-1, Insulin (Sigma), Copaxone (Teva Pharm, Israel), IgG and HSA (Omrix, Tel-Aviv, Israel). SPR analysis was assessed in 50mM Na-citrate buffer (pH 7.0) at 25°C with a flow rate of 10µl/min for 4min using either PEGylated-liposomes or control-liposomes in a final concentration of 0.2-2 mM. Regeneration of the sensor chip surface was performed by washing the chip with 1mM NaOH for 1min at a flow rate of 10µl/min. Analysis of SPR data for association, dissociation and affinity constants was carried out by BIA evaluation software (Biacore AB, Uppsala, Sweden).

**Multiple Alignments -** Multiple sequence alignment was carried out using MUSCA software (http://cbcsrv.watson.ibm.com). The following Swiss-Prot data base accession numbers were used for the multiple alignments: Human (h) FVIII (P00451), hFIX (P00740), hG-CSF (P09919), hGM-CSF (P04141), hIFN-γ (P01579), and hGLP-1 (P01275).

### 1.2 Results:

### Binding of proteins/peptides to PEGylated liposomes

We analyzed the binding of proteins and peptides to PEGylated liposomes by Surface Plasmon Resonance (SPR) measurement using a Biacore instrument (Biacoe, Uppsala, Sweden). We immobilized proteins/peptides on a CM5 sensor chip (Biacoe, Uppsala, Sweden), then injected PEGylated liposomes or control liposomes of the same size (80-110 nm) and concentration and measured and analyzed the binding of protein/peptide to the flowed intake liposomes.

PEGylated liposomes composed of POPC and DSPE-PEG2000 bind to FVIII (Fig. 1a). The binding was dependant on the PEG polymer attached to DSPE lipid since two types of control liposomes composed of POPC and POPC:DSPE did not bind to FVIII (Fig. 1a). In addition, the binding was specific to FVIII, since the PEGylated liposomes did not bind to human serum albumin (HSA) (Fig. 1b). Binding analysis of a representative curve (Fig. 1a) using a two-site binding model indicates that the PEGylated liposomes bind to two sites on FVIII with association rate constants *(Kₒₙ)* of 3.83x10⁵ and 3.37x10⁶ M-1 S-1, dissociation rate constants *(Koff)* of 1.72x10⁻³ and 6.6x10⁻³ S⁻¹ and affinity constant (K*d*) values of 1.96 nM and 4.5 nM respectively (Table 1).

Polyclonal anti human factor VIII antibodies [serum of a patient that developed anti FVIII antibodies (inhibitors)] compete with the binding of the PEGylated liposomes to both sites (Fig. 1c). Control experiment indicates that total human immunoglobulin G (IgG) did not compete with the binding of FVIII to PEGylated liposomes (data not shown). This indicates that the PEGylated liposomes specifically bind to the same protein domains as anti human factor VIII antibodies.

SPR measurements were performed with several additional recombinant and purified proteins. The following proteins were found bind to PEGylated liposomes: Factor IX (FIX), Granulocyte colony-stimulating factor (G-CSF), Granulocyte macrophage colony-stimulating factor (GM-CSF), Interferon γ and Glucagon-Like Peptide 1 (GLP-1) (Fig 2-6). In addition, we found that the following proteins do not bind to the PEGylated liposomes: HSA, IgG Insulin, Interferon α 2a, Interferon α 2b, Human growth hormone and Erythropoietin.

In addition, polyclonal anti human factor IX antibodies (Sigma) compete with the binding of the PEGylated liposomes to both sites (Fig. 7). This indicates that the PEGylated liposomes specifically bind to the same protein domains as anti human factor IX antibodies.

Amino acid sequence analysis indicate that there is a consensus sequence of 8 amino acids (S/T X-L/I/V-I/V/Q/S-S/T-X-X-E) located within the proteins that binds the PEGylated liposomes (Fig. 8a) but is absent in proteins that do not bind the PEGylated liposomes. To test the consensus sequence and the identified binding sites on FVIIII, we synthesized a peptide derived from amino acids 1783-1796 of FVIII and measured its binding to PEGylated liposomes. The peptide binds to PEGylated liposomes, but not to control POPC liposomes, with a *Kd* of 2.25 nM (Fig. 8c) which is similar to the *Kd* values of FVIII and PEGylated liposomes that were previously found (Table 1).

A summary of *Kₒₙ K_{off}* and *Kd* values of the various proteins and peptide is shown in Table 1.

However, Copaxone (Teva, Israel), a synthetic random copolymer composed of repeats of 4 amino acids (L-ala, L-glu, L-lys and L-tyr) but does not contain the consensus sequence, also binds PEGylated liposomes (Fig. 9)

**Table 1: Kinetic parameter for the binding of proteins/peptide to PEGylated liposomes.**

| Protein/peptide | *kₒₙ* (s⁻¹) | *k_{off}* (M⁻¹s⁻¹) | *K_{d}* (M) |
|---|---|---|---|
| Factor VIII (1) | 3.83x10⁵, | 1.72x10⁻³, | 1.96*10⁻⁹, |
| (2) | 3.37x10⁶ | 6.6x10⁻³ | 4.5*10⁻⁹ |
| Factor IX | 3.33*10⁶ | 0.021 | 6.3*10⁻⁹ |
| G-CSF | 4.04*10⁵ | 0.0137 | 3.39*10⁻⁸ |
| GM-CSF | 8.5*10⁵ | 0.0103 | 1.21*10⁻⁸ |
| INF-γ | 1.44*10⁶ | 4.16*10⁻³ | 2.88*10⁻⁹ |
| GLP-1 | 1.16*10⁵ | 2.21 *10⁻⁴ | 1.91*10⁻⁹ |

Association and dissociation of various proteins and a peptide to PEGliposomes were assessed, as described in "material and methods". The PEGliposome concentrations tested were 18.355nM for FVIIII, FIX, G-CSF, GM-CSF, INF-γ and 183.55pM for GLP-1. The data obtained for all the proteins were analyzed to calculate association rate constants *(kₒₙ),* dissociation rate constants *(k_{off})* and affinity constants (*K_{d}*) by BIAevaluation software.

### 2. Examples 9-10

### 2.1 Material & Methods

Formulation of FIX and G-CSF with PEGylated liposomes. PEGylated liposomes were formulated with either FIX (Octanine, Octapharma) or G-CSF (ProSpec-Tany TechnoGene Ltd; Nes Ziona, Israel) by dissolving the protein with liposome solution (one ml liposome solution/200 units of FIX and 1ml of liposome solution/10µg of G-CSF). The vial was incubated on a SRT1 roller mixer rotate at 33 rpm, amplitude 16 mm (Stuart Scientific, Redhill, UK) for 10 minutes (G-CSF) or 60 minutes (FIX), at room temperature (20-25°C).

Pharmacokinetics of liposome-formulated G-CSF and free G-CSF in mice. Two groups of C57 black mice were subcutaneously (s.c.) injected with: a) 50µl of G-CSF formulated with PEGylated liposomes (10µg/ml). b) 50µl of G-CSF dissolved in 50mM Na-citrate buffer (10µg/ml). Mice were bled from retro-orbital sinus into microcentrifuge tubes containing sodium citrate (20 mM final concentration) at various times post injection and plasma was separated by centrifugation at 2,700 x g for 10 minutes at 4 °C. G-CSF concentrations in mouse plasma was measured by ELISA (G-CSF DouSet ELISA kit, R&D) according to manufacturer instruction

Pharmacokinetics of PegLip-FIX and free FIX in hemophilic mice. Three groups of C57BL mice were injected IV into the tail vein with 50 µl of: a) 200 units/ml PegLip-FIX. b) un-formulated FIX. c) buffer. Mice were bled from retro-orbital sinus into microcentrifuge tubes containing sodium citrate (20 mM final concentration) at various times post injection and plasma was separated by centrifugation at 2,700 x g for 10 minutes at 4 °C. FIX activity in mouse plasma was measured by one-stage clotting assay (using Stago reagents and ST4 clotting machine) according to manufacturer's instruction. Since C57BL mice have endogenous FIX activity in their plasma, this endogenous activity (as measured in the control mice) was subtracted from the activity measured at each time-point in the treated groups.

### Pharmacokinetics analysis

Pharmacokinetic parameters (half life and AUC) were analyzed by computer software (RSTRIP, MicroMath Inc.).

### Statistic analysis

Student's t-test.

### 2.2 Results

### Pharmacokinetics of liposome-formulated proteins in mice

Pharmacokinetic parameters of free and liposome formulated FIX and G-CSF were measured in mice. The results presented in Tables 2-3 indicate that the half-life and area under the curve of the proteins formulated with PEGylated liposomes were higher than that of free proteins.

**Table 2: Pharmacokinetic parameters following SC injection of liposome-formulated G-CSF (PegLip-G-CSF or free G-CSF into mice**

| | T=1.75hr | T=3.75hr | T=5.75hr | T=7.75hr | T=9.33hr | HL (hr.) | AUC (pg*h/ml) |
|---|---|---|---|---|---|---|---|
| PegLip-G-CSF | 58150.72 ±6299.95 | 56023.32 ±7372.35 | 48901.71 ±8991.09 | 24318.43 ±6512.68 | 14006.75 ±4278.52 | 5.038 0.896 | 434386.17 ±55926.46 |
| | T=2hr | T=4hr | T=6hr | T=8hr | T=9.58hr | | |
| G-CSF | 62419.23 ±5212.47 | 50556.82 ±6928.12 | 37141.44 ±6068.82 | 12705.32 ±2780.72 | 5735.84 ±284.49 | 3.181 ±0.354 | 420746.83 ±40361.42 |

G-CSF was reconstituted with 50mM Na-citrate buffer pH 7 or formulated with PEGylated liposomes and injected (50 µl) s.c. into mice (6 mice in a group). Human G-CSF concentration (µg/ml) in the plasma was measured by an Enzyme Linked Immunosorbent Assay (Elisa). Pharmacokinetic parameters were calculated for each mouse. Values are means ± standard deviation. Student t-test analysis for half-life ofPegLip-G-CSF *versus* G-CSF, P<0.002.

**Table 3: Pharmacokinetic parameters following IV injection of Factor IX or PEGylated liposome-formulated Factor IX (PegLip-FIX) into mice.**

| | T=10min | T=4.83 hr | T=19.83hr | HL (hr.) | AUC (pg*h/ml) |
|---|---|---|---|---|---|
| PegLip-FIX | 3.78±0.51 | 0.77±0.41 | 0.092±0.1 | 11.72 | 2.02 |
| | T=10mm | T=5hr | T=29hr | | |
| FIX | 3.85±0.39 | 0.63±0.31 | 0.081±0.078 | 10.96 | 1.85 |

Pharmacokinetic parameters were calculated for each mouse. Values are means ± standard deviation. Student t-test analysis for half-life of PegLip-FIX *versus* FIX, *P*<0.15.

### 3. Example 11

### 3.1 Pharmacokinetics and biological activity of FVIII formulated in-vivo with PEGylated liposomes in hemophilic mice

Pharmacokinetics and biological activity of factor VIII that was formulated *in-vivo* with PEGylated ilposomes by injection of liposomes 1 hour after the injection of unformulated factor VIII, was measured in hemophilic mice. Hemophilic mice were injected with: a) free (unformulated) FVIII. b) Free FVIII and one hour later a second injection of PEGylated liposomes. The mice were bled at various times post-injection FVIII activity was measured by a clotting assay. In order to test in-vivo efficacy of liposome-formulated FVIII in stopping bleeding and compare it to that of free FVIII tails of the injected mice were cut several times post injection and the survival of the injected mice was measured.

The results presented in Table 4 and Figure 10 indicate that the half-life and area under the curve of factor VIII that was formulated in-vivo with PEGylated liposomes were higher than that of free FVIII. Accordingly, the survival of mice injected with FVIII and 1 hour later with liposomes were significantly higher.

**Table 4: Factor VIII activity (IU/ml) and pharmacokinetic parameters following injection of FVIII or FVIII and 1 hour later PEGylated liposomes into hemophilic mice**

| **Injection material** | **Time Post injection** | **T=10min** | **T=4.6h** | **T=8.3h** | **T=24.6 h** | **T=28.8 h** | **Half Life** | **Area under the curve** |
|---|---|---|---|---|---|---|---|---|
| **FVIII and liposomes (Injected 1 hour later)** | Average (u/ml) | 2.03 | 1.35 | 0.975 | 0.66 | 0.61 | 14.5 | 28.8 |
| **FVIII** | Average (u/ml) | 2.17 | 1.4 | 0.95 | 0.46 | 0.41 | 9.3 | 24.0 |

Recombinant FVIII (Kogenate-FS, Bayer) was reconstituted with water and injected (40 µl) into the tail vein of hemophilic mice (10 mice in each group). One our later, PEGylated liposomes (9% lipids, w/v) were injected i.v. (40 µl) into one group of mice. FVIII activity was measured on pool plasma sample of each time-point by a one-stage clotting assay. Pharmacokinetic parameters (half life and AUC) were analyzed by a computer program (RSTRIP, MicroMath Inc.).

### 4. Example 12

The binding of FVIII to liposomes composed of POPC and carbamate-linked uncharged lipopolymer was compared to the binding of FVIII to liposomes composed of POPC and DSPE-PEG by real time interaction analysis. The schematic structure of carbamate-linked uncharged lipopolymer: mPEG aminopropanediol disrearoyl (DS-c-PEG) and 1,2 distearoyl-sn-glycero-3-phosphatidylethanolamine-N-methoxy polyethylene glycol (DSPE-PEG) are shown below.

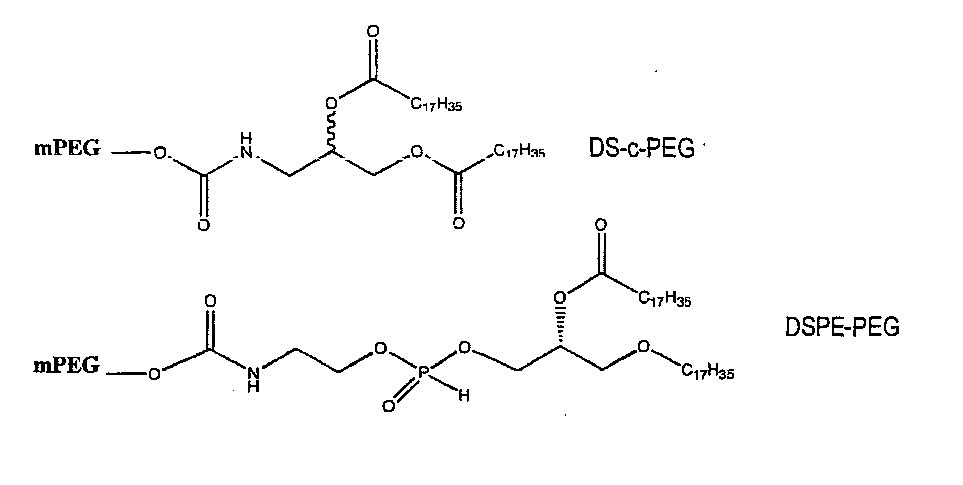

The results are presented in Fig. 11 and show that both types of liposomes interacts with FVIII.

### 5. Example 13.

Coagulation factor VIIa is generally used to treat hemophilia patients with inhibitors and to stop trauma bleeding (e.g. war injuries, car accidents). Pharmacokinetics of factor VIIa, formulated with PEGylated liposomes, was measured in rats. Sprague Dawley (SD) rats (180 g) rats were injected with 36µg/rat of free (unformulated) FVIIa (Nova Nordisk) or FVIIa formulated with PEGylated liposomes. The rats were bled at various times post-injection and FVIIa activity was measured by a clotting assay (Stago). The results presented in Tables 5 and 6 indicate that the half-life and area under the curve of factor VIIa that was formulated in-vivo with PEGylated liposomes were higher than that of free FVIIa.

**Table 5: Factor VIIa activity (IU/ml) and pharmacokinetics following injection of FVlla or FVlla formulated with pegylated liposomes into rats**

| **Injected material** | **Time post-injection** | **T=10m in** | **T=3 hrs** | **T=7 hrs** | **T=24 hrs** | **T=30 hrs** | **Half Life (HL) (h)** | **Area under the curve (AUC)** |
|---|---|---|---|---|---|---|---|---|
| PEGLi p-rFVIIa (n=4) | Average (U/ml) ± SD | 573 ± 255 | 38 ± 8 | 7 ± 1 | 1.6 ± 1 | 0.5 ± 0.2 | 3.08 | 2013 |
| rFVIIa (n=4) | Average (U/ml) ±SD | 706 ± 45 | 45 ± 3 | 4.6 ± 1 | 0.1 ± 0.1 | 0 | 1.86 | 1411 |

**Table 6: Factor VIIa activity (IU/ml) and pharmacokinetics following injection of FVIIa or FVIIa formulated with pegylated liposomes into rats (pooled samples)**

| **Injected material** | **T~10min** | **T~3 hrs** | **T~7 hrs** | **T~23 hrs** | **T~31 hrs** | **Half Life (HL) (h)** | **Area under the curve (AUC)** |
|---|---|---|---|---|---|---|---|
| PEGLip-rFVIIa (n=3) | 287 | 22.6 | 8.7 | 1.6 | 1.2 | 3.84 | 1019 |
| rFVIIa (n=3) | 319 | 27 | 5 | 0 | 0 | 1.15 | 513 |

## Claims

1. A pharmaceutical composition for parenteral administration comprising a therapeutically effective amount of a protein or polypeptide non-covalently bound to a colloidal particle, said particle comprising approximately 1-20 mole percent of an amphipathic lipid derivatized with a biocompatible hydrophilic polymer,
wherein said protein or polypeptide is selected from the group consisting of:(a) Factor VIIa, granulocyte colony-stimulating factor (G-CSF), granulocyte macrophage colony-stimulating factor (GM-CSF), interferon γ, glucagon-*like* peptide 1 (GLP-1) and Copaxone; and(b) proteins or polypeptides that include a consensus sequence of serine/threonine-X-leucine/iscleucine/valine-isoleucine/valine/glutamine/serine-serine/threonine-X-X-glutamic acid, where X may be any amino acid;
wherein said protein or polypeptide is not Factor VIII (FVIII),
and wherein said protein or polypeptide is not encapsulated in said colloidal particles.

2. The pharmaceutical composition of Claim 1 wherein said colloidal particles are substantially neutral and said polymer carries substantially no net charge.

3. The pharmaceutical composition of Claim 1 or Claim 2 wherein said amphipathic lipid is a phospholipid from natural or synthetic sources.

4. The pharmaceutical composition of Claim 3 wherein said amphipathic lipid is phosphatidylethanolamine (PE).

5. The pharmaceutical composition of Claim 1 or Claim 2 wherein said amphipathic lipid is a carbamate-linked uncharged lipopolymer.

6. The pharmaceutical composition of Claim 5 wherein said amphipathic lipid is aminopropanediol distearoyl (DS).

7. The pharmaceutical composition of any of the preceding claims wherein said colloidal particles further comprise a second amphipathic lipid obtained from either natural or synthetic sources.

8. The pharmaceutical composition of Claim 7 wherein said second amphipathic lipid is phosphatidylcholine.

9. The pharmaceutical composition of any of the preceding claims wherein said biocompatible hydrophilic polymer is polyethylene glycol.

10. The pharmaceutical composition of Claim 9 wherein the polyethylene glycol has a molecular weight of between about 500 to about 5000 daltons.

11. The pharmaceutical composition of any of Claims 1 to 10 wherein the polypeptide is Copaxone and the composition may be used for the treatment of a disease selected from multiple sclerosis, diabetic neuropathy, senile dementia, Alzheimer's disease, Parkinson's Disease, facial nerve (Bell's) palsy, glaucoma, Huntington's chorea, amyotrophic lateral sclerosis, status epilepticus, non-arteritic optic neuropathy and vitamin deficiency.

12. The pharmaceutical composition of any of Claims 1, to 10 wherein the polypeptide is Factor VIIa and the composition may be used in hemophilia patients with inhibitors and for the treatment of trauma bleeding.

13. Use of a colloidal particle in the preparation of a pharmaceutical composition for parenteral administration for treatment of a patient suffering from a disease, said composition comprising a therapeutically effective amount of a protein or polypeptide non-covalently bound to a colloidal particle, said colloidal particle comprising approximately 1-20 mole percent of an amphipathic lipid derivatized with a biocompatible hydrophilic polymer,
wherein said protein or polypeptide is selected from the group consisting of: (a) Factor VIIa, granulocyte colony-stimulating factor (G-CSF), granulocyte macrophage colony-stimulating factor (GM-CSF), interferon γ, glucagon-like peptide 1 (GLP-1) and Copaxone; and(b) proteins or polypeptides that include a consensus sequence of serine/threonine-X-leucine/isolcucine/valine-isoleucine/valinelglutaminelserine-serine/threonine-X-X-glutamic acid, where X may be any amino acid;
wherein said protein or polypeptide is not Factor VIII (FVIII),
and wherein said protein or polypeptide is not encapsulated in said colloidal particles.

14. The use of claim 13 wherein said disease is hemophilia.

15. The use of Claim 13 or Claim 14 wherein the colloidal particle is a liposome.

16. Use of colloidal particles and a protein or polypeptide in the preparation of a pharmaceutical composition for parenteral administration for treatment of a patient suffering from a disease, wherein said pharmaceutical composition comprises a therapeutically effective amount of the protein or polypeptide and the colloidal particles, the colloidal particles comprising approximately 1-20 mole percent of an amphipathic lipid derivatized with a biocompatible hydrophilic polymer, wherein said protein or polypeptide is selected from the group consisting of;
(a) proteins or polypeptides capable of externally binding said colloidal particles;
(b) proteins or polypeptides capable of binding polymers of the polyalkylether, polylactic and polyglycolic acid families; and
(c) proteins or polypeptides that include a consensus sequence of serine/threonine-X-leucine/isoleucine/valine-isoleucine/valine/glutamine/serine-serine/theonine-X-X-glutamic acid, where X may be any amino acid;
wherein the protein or polypeptide is not encapsulated in the colloidal particles,
and wherein the colloidal particles and the protein or polypeptide are separate.

17. The use of Claim 16 wherein the protein or polypeptide is not Factor VIII (FVIII).

## Patentansprüche

1. Pharmazeutische Zusammensetzung zur parenteralen Verabreichung, umfassend eine therapeutisch wirksame Menge eines nicht kovalent an ein kolloidales Partikel gebundenen Proteins oder Polypeptids, wobei das Partikel ungefähr 1 bis 20 Mol-% eines amphipathischen Lipids umfasst, das mit einem biokompatiblen hydrophilen Polymer derivatisiert ist,
wobei das Protein oder Polypeptid ausgewählt wird aus der Gruppe, die besteht aus:
(a) Faktor Vlla, Ganulozyten-koloniestimulierendem Faktor (G-CSF), Granulozyten-Makrophagen-koloniestimulierendem Faktor (GM-CSF), Interferon γ, Glucagon-artigem Peptid 1 (GLP-1) und Copaxon; und
(b) Proteinen oder Polypeptiden, die eine Konsensus-Sequenz von Serin/Threonin-X-Leucin/Isoleucin/Valin-Isoleucin/Valin/Glutamin/Serin-Serin/Threonin-X-X-Glutaminsäure umfassen, wobei X eine beliebige Aminosäure sein kann;
wobei das Protein oder Polypeptid nicht Faktor VIII (FVIII) ist,
und wobei das Protein oder Polypeptid nicht in den kolloidalen Partikeln eingekapselt ist,

2. Pharmazeutische Zusammensetzung nach Anspruch 1 , wobei die kolloidalen Partikel im Wesentlichen neutral sind und das Polymer im Wesentlichen keine Nettoladung trägt.

3. Pharmazeutische Zusammensetzung nach Anspruch 1 oder 2, wobei das amphipathische Lipid ein Phospholipid aus natürlichen oder synthetischen Quellen ist.

4. Pharmazeutische Zusammensetzung nach Anspruch 3, wobei das amphipathische Lipid Phosphatidylethanolamin (PE) ist.

5. Pharmazeutische Zusammensetzung nach Anspruch 1 oder 2, wobei das amphipathische Lipid ein Carbamat-gebundenes ungeladenes Lipopolymer ist.

6. Pharmazeutische Zusammensetzung nach Anspruch 5, wobei das amphipathische Lipid Aminopropandioldistearoyl (DS) ist.

7. Pharmazeutische Zusammensetzung nach einem der vorstehenden Ansprüche, wobei die kolloidalen Partikel darüber hinaus ein zweites amphipathisches Lipid umfassen, das entweder aus natürlichen oder synthetischen Quellen erhalten wird.

8. Pharmazeutische Zusammensetzung nach Anspruch 7, wobei das zweite amphipathische Lipid Phosphatidylcholin ist.

9. Pharmazeutische Zusammensetzung nach einem der vorstehenden Ansprüche, wobei das biokompatible hydrophile Polymer Polyethylenglykol ist.

10. Pharmazeutische Zusammensetzung nach Anspruch 9, wobei das Polyethylenglykol ein Molekulargewicht von zwischen etwa 500 bis etwa 5000 Dalton aufweist.

11. Pharmazeutische Zusammensetzung nach einem der Ansprüche 1 bis 10, wobei das Polypeptid Copaxon ist und die Zusammensetzung für die Behandlung einer Erkrankung verwendet werden kann, die ausgewählt wird aus Multipler Sklerose, diabetischer Neuropathie, seniler Demenz, Alzheimer-Krankheit, Parkinson-Krankheit, Gesichtsnerv-Lähmung ((Bell's palsy), Glaukom, Chorea Huntington, amyotropher Lateralsklerose, Status epileptikus, nicht-arteritischer optischer Neuropathie und Vitaminmangel.

12. Pharmazeutische Zusammensetzung nach einem der Ansprüche 1 bis 10, wobei das Polypeptid Faktor VIIa ist und die Zusammensetzung bei Hämophilie-Patienten mit Inhibitoren und für die Behandlung von Traumablutungen verwendet werden kann.

13. Verwendung eines kolloidalen Partikels bei der Herstellung einer pharmazeutischen Zusammensetzung zur parenteralen Verabreichung zur Behandlung eines Patienten, der an einer Erkrankung leidet, wobei die Zusammensetzung eine therapeutisch wirksame Menge eines nichtkovalent an ein kolloidales Partikel gebundenen Proteins oder Polypeptids umfasst, wobei das kolloidale Partikel ungefähr 1 bis 20 Mol-% eines amphipathischen Lipids umfasst, das mit einem biokompatiblen hydrophilen Polymer derivatisiert ist,
wobei das Protein oder Polypeptid ausgewählt wird aus der Gruppe, die besteht aus:
(a) Faktor VIIa, Ganulozyten-koloniestimulierendem Faktor (G-CSF), Granulozyten-Makrophagen-koloniestimulierendem Faktor (GM-CSF), Interferon γ, Glucagon-artigem Peptid 1 (GLP-1) und Copaxon; und
(b) Proteinen oder Polypeptiden, die eine Konsensus-Sequenz aus Serin/Threonin-X-Leucin/Isoleucin/Valin-Isoleucin/Valin/Glutamin/Serin-Serin/Threonin-X-X-Glutaminsäure umfassen, wobei X eine beliebige Aminosäure sein kann;
wobei das Protein oder Polypeptid nicht Faktor VIII (FVIII) ist,
und wobei das Protein oder Polypeptid nicht in den kolloidalen Partikeln eingekapselt ist.

14. Verwendung nach Anspruch 13, wobei die Erkrankung Hämophilie ist.

15. Verwendung nach Anspruch 13 oder Anspruch 14, wobei das kolloidale Partikel ein Liposom ist.

16. Verwendung von kolloidalen Partikeln und eines Proteins oder Polypeptids bei der Herstellung einer pharmazeutischen Zusammensetzung zur parenteralen Verabreichung zur Behandlung eines Patienten, der an einer Erkrankung leidet, wobei die pharmazeutische Zusammensetzung eine therapeutisch wirksame Menge des Proteins oder Polypeptids und der kolloidalen Partikel umfasst, wobei die kolloidalen Partikel ungefähr 1 bis 20 Mol-% eines amphipathischen Lipids umfassen, das mit einem biokompatiblen hydrophilen Polymer derivatisiert ist, wobei das Protein oder Polypeptid ausgewählt wird aus der Gruppe, die besteht aus:
(a) Proteinen oder Polypeptiden, die zur externen Bindung der kolloidalen Partikel fähig sind;
(b) Proteinen oder Polypeptiden, die zur Bindung von Polymeren der Polyalkylether-, Polymilchsäure- und Polyglykolsäure-Familien fähig sind; und
(c) Proteinen oder Polypeptiden, die eine Konsensus-Sequenz aus Serin/Threonin-X-Leucin/Isoleucin/Valin-Isoleucin/Valin/Glutamin/Serin-Serin/Threonin-X-X-Glutaminsäure beinhalten, wobei X eine beliebige Aminosäure sein kann;
wobei das Protein oder Polypeptid nicht in den kolloidalen Partikeln eingekapselt ist,
und wobei die kolloidalen Partikel und das Protein oder Polypeptid getrennt vorliegen.

17. Verwendung nach Anspruch 16, wobei das Protein oder Polypeptid nicht Faktor VIII (FVIII) ist.

## Revendications

1. Composition pharmaceutique pour l'administration par voie parentérale comprenant une quantité efficace, du point de vue thérapeutique, d'une protéine ou d'un polypeptide lié(e) de manière non covalente à une particule colloïdale, ladite particule comprenant approximativement 1-20 % en moles d'un lipide amphipathique transformé en dérivé avec un polymère hydrophile biocompatible,
dans laquelle ladite protéine ou ledit polypeptide est choisi (e) dans l'ensemble constitué par : (a) le Facteur VIIa, le facteur stimulant les colonies granulocytaires (G-CSF), le facteur stimulant les colonies de macrophages-granulocytes (GM-CSF), l'interféron γ, le peptide 1 analogue au glucagon (GLP-1) et la Copaxone ; et (b) les protéines ou polypeptides qui contiennent une séquence consensus sérine/thréonine-X-leucine/isoleucine/valine-isoleucine/valine/glutamine/sérine-sérine/thréonine-X-X-acide glutamique, où X peut être n'importe quel acide aminé ;
dans laquelle ladite protéine ou ledit polypeptide n'est pas le Facteur VIII (FVIII),
et dans laquelle ladite protéine ou ledit polypeptide n'est pas encapsulé(e) dans lesdites particules colloïdales.

2. Composition pharmaceutique selon la revendication 1, dans laquelle lesdites particules colloïdales sont pratiquement neutres et ledit polymère ne porte pratiquement pas de charge nette.

3. Composition pharmaceutique selon la revendication 1 ou la revendication 2, dans laquelle ledit lipide amphipathique est un phospholipide provenant de sources naturelles ou synthétiques.

4. Composition selon la revendication 3, dans laquelle ledit lipide amphipathique est la phosphatidyléthanolamine (PE).

5. Composition pharmaceutique selon la revendication 1 ou la revendication 2, dans laquelle ledit lipide amphipathique est un lipopolymère non chargé lié à un carbamate.

6. Composition pharmaceutique selon la revendication 5, dans laquelle ledit lipide amphipathique est l'aminopropanediol-distéaroyle (DS).

7. Composition pharmaceutique selon l'une quelconque des revendications précédentes, dans laquelle lesdites particules colloïdales comprennent en outre un deuxième lipide amphipathique obtenu à partir de sources soit naturelles soit synthétiques.

8. Composition pharmaceutique selon la revendication 7, dans laquelle ledit deuxième lipide amphipathique est la phosphatidylcholine.

9. Composition pharmaceutique selon l'une quelconque des revendications précédentes, dans laquelle ledit polymère hydrophile biocompatible est le polyéthylèneglycol.

10. Composition pharmaceutique selon la revendication 9, dans laquelle le polyéthylèneglycol a une masse moléculaire comprise entre environ 500 et environ 5000 daltons.

11. Composition pharmaceutique selon l'une quelconque des revendications 1 à 10, dans laquelle le polypeptide est la Copaxone et la composition peut être utilisée pour le traitement d'une maladie choisie parmi la sclérose en plaques, la neuropathie diabétique, la démence sénile, la maladie d'Alzheimer, la maladie de Parkinson, la paralysie faciale (de Bell), le glaucome, la chorée de Huntington, la sclérose latérale amyotrophique, l'état de mal convulsif, la neuropathie optique non-artérielle et la carence vitaminique.

12. Composition pharmaceutique selon l'une quelconque des revendications 1 à 10, dans laquelle le polypeptide est le Facteur VIIa et la composition peut être utilisée chez des patients hémophiles avec des inhibiteurs et pour le traitement de saignements traumatiques.

13. Utilisation d'une particule colloïdale dans la préparation d'une composition pharmaceutique pour l'administration parentérale pour le traitement d'un patient souffrant d'une maladie, ladite composition comprenant une quantité efficace, du point de vue thérapeutique, d'une protéine ou d'un polypeptide lié(e) de manière non covalente à une particule colloïdale, ladite particule colloïdale comprenant approximativement 1-20 % en moles d'un lipide amphipathique transformé en dérivé avec un polymère hydrophile biocompatible,
dans laquelle ladite protéine ou ledit polypeptide est choisi(e) dans l'ensemble constitué par : (a) le Facteur VIIa, le facteur stimulant les colonies granulocytaires (G-CSF), le facteur stimulant les colonies de macrophages-granulocytes (GM-CSF), l'interféron γ, le peptide 1 analogue au glucagon (GLP-1) et la Copaxone ; et (b) les protéines ou polypeptides qui contiennent une séquence consensus sérine/thréonine-X-leucine/isoleucine/valine-isoleucine/valine/glutamine/sérine-sérine/thréonine-X-X-acide glutamique, où X peut être n'importe quel acide aminé ;
dans laquelle ladite protéine ou ledit polypeptide n'est pas le Facteur VIII (FVIII),
et dans laquelle ladite protéine ou ledit polypeptide n'est pas encapsulé(e) dans lesdites particules colloïdales.

14. Utilisation selon la revendication 13, dans laquelle ladite maladie est l'hémophilie.

15. Utilisation selon la revendication 13 ou la revendication 14, dans laquelle la particule colloïdale est un liposome.

16. Utilisation de particules colloïdales et d'une protéine ou d'un polypeptide dans la préparation d'une composition pharmaceutique pour l'administration parentérale pour le traitement d'un patient souffrant d'une maladie, dans laquelle ladite composition pharmaceutique comprend une quantité efficace, du point de vue thérapeutique, de la protéine ou du polypeptide et des particules colloïdales, les particules colloïdales comprenant approximativement 1-20 % en moles d'un lipide amphipathique transformé en dérivé avec un polymère hydrophile biocompatible, dans laquelle ladite protéine ou ledit polypeptide est choisi dans l'ensemble constitué par :
(a) les protéines ou polypeptides capables de se lier à la partie externe desdites particules colloïdales ;
(b) les protéines ou polypeptides capables de se lier à des polymères des familles des polyalkyléthers, poly(acide lactique) et poly(acide glycolique) ; et
(c) les protéines ou polypeptides qui contiennent une séquence consensus sérine/thréonine-X-leucine/isoleucine/valine-isoleucine/valine/glutamine/sérine-sérine/thréonine-X-X-acide glutamique, où X peut être n'importe quel acide aminé ;
dans laquelle la protéine ou le polypeptide n'est pas encapsulé(e) dans les particules colloïdales,
et dans laquelle les particules colloïdales et la protéine ou le polypeptide sont séparé(e)s.

17. Utilisation selon la revendication 16, dans laquelle la protéine ou le polypeptide n'est pas le Facteur VIII (FVIII).
